# EUROPEAN PATENT APPLICATION

(11) **EP 1 457 483 A1**
(43) Date of publication of application: **15.09.2004**
(21) Application number: 03005513.1
(22) Date of filing: 11.03.2003
(51) Int. Cl.: C07C 237/10, A61K 48/00, C12N 15/87

(54) **Cationic lipids with serine backbone**

(71) Applicant: Ruhr-Universität Bochum, 44801 Bochum (DE); KTB Tumorforschungsgesellschaft mbH, 79106 Freiburg (DE)
(72) Inventor: Lenssen, Karl, Christian, 42555 Velbert (DE); Jantscheff, Peter, 79211 Denzlingen (DE); Kiedrowski, Günter, 44797 Bochum (DE); Massing, Ulrich, 79249 Merzhausen (DE)
(74) Representative: Helbing, Jörg, Dr. Dipl.-Chem.

(57) **Abstract**

The present invention provides cationic lipids with serine backbone, a composition for transferring biologically active molecules into cells and/or tissues comprising said cationic lipids, a process for the manufacture of said lipids, the use of said lipids as constituent of a transfection agent and a method for transferring biologically active molecules into cells and/or into tissues or for gene therapy.

## Description

The present invention provides cationic lipids with serine backbone, a composition for transferring biologically active molecules into cells and/or tissues comprising said cationic lipids, a process for the manufacture of said lipids, the use of said lipids as constituent of a transfection agent and a method for transferring biologically active molecules into cells and/or into tissues or for gene therapy.

### Background of the Invention

Gene therapy is a promising strategy for treating acquired or inherited genetic diseases such as cancer or cystic fibrosis. The fundamental hurdle in the transfer of *gene therapy* from the experimental stage to clinical practice is the development of vectors to deliver genetic material (therapeutic genes) to the appropriate cells in a specific, efficient, and safe manner. This problem of "drug delivery," where the drug is a gene, is particularly challenging for genes which are large and complex and which require targeting to the nuclei of cells. Most of the vectors currently in use for clinical gene therapy trials are based on attenuated or modified versions of viruses. Although efficient, there are serious safety problems associated with viral vectors including possible activation of the patients immune system, risk of infection with traces of the wild type virus or insertion mutagenesis. These risks were dramatically underscored by the death of an 18-year old man in an adenovirus based gene therapy study in 1999.
A promising alternative is gene transfer mediated by cationic lipids (lipofection): Cationic lipids have the advantage of directly interacting with the polyanion DNA, thereby forming lipid-DNA complexes (Massing, U. Lipid Technology 13(3):57-60 (2001)). These mostly positively charged complexes, or lipoplexes, are supposed to bind to and be endocytosed by (negatively charged) cells. The transferred genetic material (DNA, RNA, or oligonucleotides) then has to escape lysosomal degradation and to enter the nucleus to reach its target. Lipofection bears advantages over viral transfection: There is no restriction on the size of the therapeutic gene and no risk of immunogenicity or infection. Additionally, cationic lipids can be manufactured in large quantities with relatively little effort. However, in contrast to viral gene transfer, the efficiency of lipofection is still poor and the mechanism of lipofection is only partly understood.
Such cationic lipids are known in the art. E.g. WO 01/57064 and Fichert, T. et al., Bioorg. Med. Chem. Letters, 10:787-791 (2000) disclose a cationic amphiphile consisting of a cholesterol or diacylglycerol moiety serving as the lipid anchor, a spacer group and a (poly)amine based cationic head group, which forms part of a lipid mixture or a liposome. Said lipid mixture or liposome may additionally contain a helper lipid. However, the synthesis of the cationic lipids described here is quite cumbersome, it requires protecting groups, and is carried out in solution phase. It is desirable to have alternative lipid anchors at hand which are easier to synthesise from readily available starting materials e.g. by means of solid phase synthesis not involving the use of protecting groups. The applicability of solid phase synthesis would also facilitate the implementation of a rapid screening process in order to find those lipids which offer optimum efficiency for the transfer of a biologically active molecule into a cell.
DE 199 25 143 describes as an alternative liposomal vector complexes (comprising a nucleic acid sequence of any length, a cationic carrier, and lipids and phospholipids forming a liposome), a process for their preparation and their use in the preparation of a drug for prophylactics or therapy of a disease. Thus, this alternative is limited to liposomes where the cationic carrier is present as an additional component. The latter has to be purchased or synthesised and requires an additional step in the preparation of the liposomal vector complexes. This also renders a screening process for finding the lipid mixture with optimum properties more laborious. By the use of a cationic lipid this cationic carrier could be omitted.
DOPE (N,N-dioleoyl phophatidylethnolamine) alone or in a mixture with DOTAP (N-(1(2,-dioleyoyl)propyl)-N,N,N-trimethylammonium chloride) and other helper lipids are known to form lipoplexes suitable for gene delivery (Hirsch-Lerner, D. and Barenholz, Y., Biochim. Biophys. Acta, 1461:47-57 (1999) and 1370:17-30 (1998); De Olivera, M.C. et al., Biochim. Biophys. Acta 1372:301-310 (1998)).
Finally, DOTAP analogues and their use in lipofection is described in Regelin, A.E. et al., BBA 1464:151-164 (2000) and in Massing, U. et al., Chem. Phys. Lipids 105:189-191 (2000).
On the other hand, JP-A-01233264 discloses a quaternary ammonium salt compound of the formula 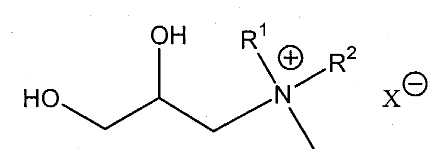 wherein R¹ and R² are saturated C₈₋₁₈ alkyl groups and X is halogen which can be utilised as germicide, e.g., for washing tableware and disinfecting livestock barns. JP-A-02223515 utilises the above quaternary ammonium compounds (wherein R¹ and R² are saturated C₈₋₁₄ alkyl groups and X is halogen) as antifungal agent.
PCT/EP02/11156 (published April 2003) discloses that the above quaternary ammonium compounds possess a transfection behaviour superior to that of DOTAP. Finally, JP-A-02225490 discloses quaternary ammonium salts of glucose or methyl glycoside (such as (6-deoxy-D-glucopyran-6-yl)didecylmethylammonium chloride) which are useful as a germicide, e.g., for dish-washing and cattle stall sterilisation.

### Summary of the Invention

By utilizing a synthetic high throughput screening approach the relationship between structure and transfection efficiency of a new class of cationic lipids for gene transfer was studied. It was found that cationic lipids with a serine backbone possess superior transfection properties as compared to DOTAP and are also suitable for transfection of cells difficult to transfect such as dendritic cells.

In particular, a library of fifty different cationic lipids based on a L-serine scaffold was synthesised by combinatorial solid phase chemistry and tested for reporter gene (GFP-luciferase) transfer in three combinations without and with two different helper lipids at eight different lipid:DNA-charge ratios using a fully automated transfection and screening method on COS-7 cells.
The lipids display striking high transfection efficiencies (up to 572.000 RLU). In dendritic cells (DC) the lipids showed lower but reasonable transfection efficiencies (300 - 570 RLU).The invention thus provides
(1) a lipid having the following formula (I) 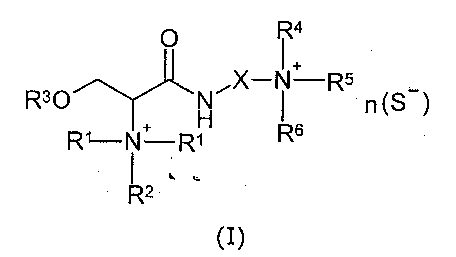 wherein
   R¹ are same or different, optionally substituted and/or unsaturated alkyl residues;
   R² and R⁶ are independently selected from a free valency (i.e., R² or R⁶ being a fee electron pair = absent), a hydrogen atom and an optionally substituted and/or optionally unsaturated alkyl residue;
   R³ is selected from a hydrogen atom, an optionally substituted and/or unsaturated and/or heteroatom-containing alkyl residue, an optionally substituted and/or unsaturated acyl residue, and a suitable protecting group;
   R⁴ and R⁵ are independently selected from a hydrogen atom, an optionally substituted and/or unsaturated and/or heteroatom-containing alkyl residue, an optionally substituted and/or unsaturated acyl residue, an optionally derivatized amino acid or polypeptide residue and a suitable protecting group or R⁴ and R⁵ together with the nitrogen atom forms an optionally substituted heterocyclic ring;
   X is an optionally substituted and/or unsaturated and/or heteroatom-containing alkylene residue;
   n(S⁻) represents (the required) biologically and/or pharmaceutically suitable counter ion(s);
(2) a composition for transferring biologically active molecules into cells and/or tissues comprising
   (a) at least one lipid as defined in (1) above; and
   (b) at least one biologically active molecule;
(3) a process for the manufacture of the lipid as defined in (1) above which 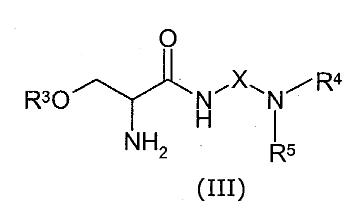 comprises reacting a compound of formula (III)
   under conditions suitable for carrying out a reductive amination with a compound of formula (IV) 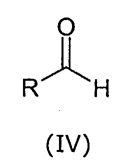 wherein all variables are as defined in (1) above, and R has the same meaning as R¹ having one CH₂ (methylene) unit less than R¹;
(4) the use of the lipid as defined in (1) above
   (i) as a transfection agent, which optionally further comprises a helper compound/lipid for transferring a biologically active molecule into cells and/or tissues *in vitro,* or
   (ii) for preparing an agent, which optionally further comprises a helper compound/lipid for transferring a biologically active molecule into cells and/or tissues *in vivo* including, but not limited to, an agent for gene therapy; and
(5) a method for transferring biologically active molecules into cells and/or into tissues in vivo and in vitro, or for gene therapy, which method comprises contacting the cells or tissues, or the subject in need of the gene therapy with a composition comprising a cationic lipid of formula (I) as defined in (1) above.

### Description of the Figures

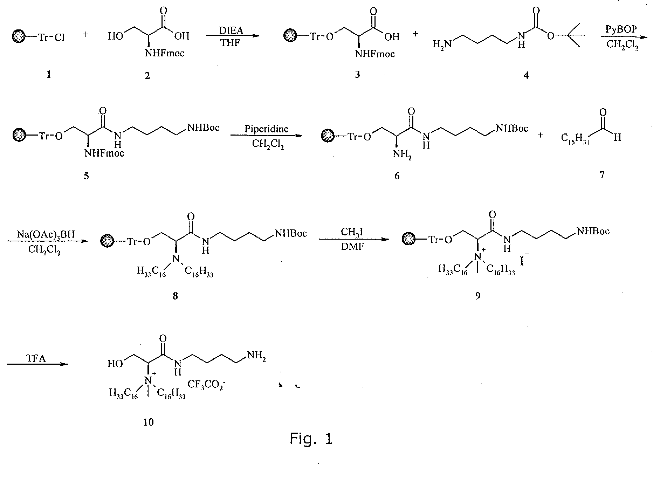

Figure 1: Combinatorial solid-phase synthesis of lipids with L-serine-scaffold. Schematic outline of general synthetic route for the preparation of the cationic MS-lipid library. Abbreviations: Boc: tert-butoxycarbonyl, (Boc)₂O: di-tert-butyl pyrocarbonate, DIEA: *N,N*-diisopropylethylamine, DMF: *N,N*-dimethylformamide, Fmoc: 9-fluorenylmethoxycarbonyl, PyBOP: benzotriazol-1-yl-oxytripyrrolidinophosphonium hexafluorophophate, TFA: trifluoroacetic acid, THF: tetrahydrofuran.

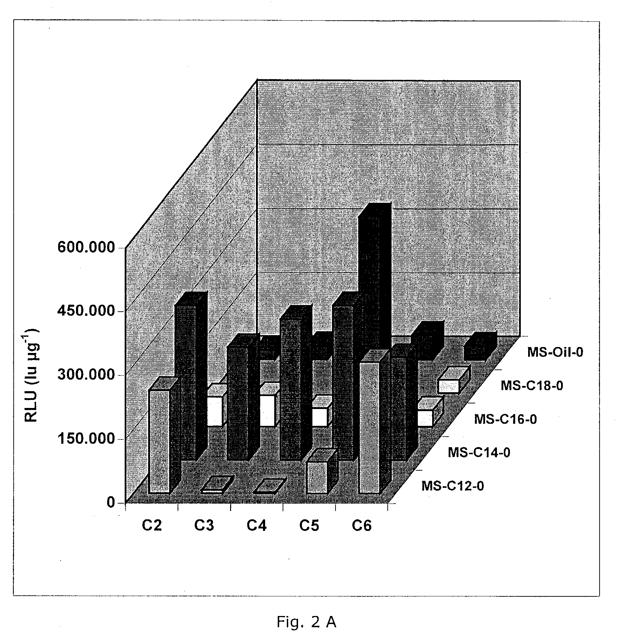

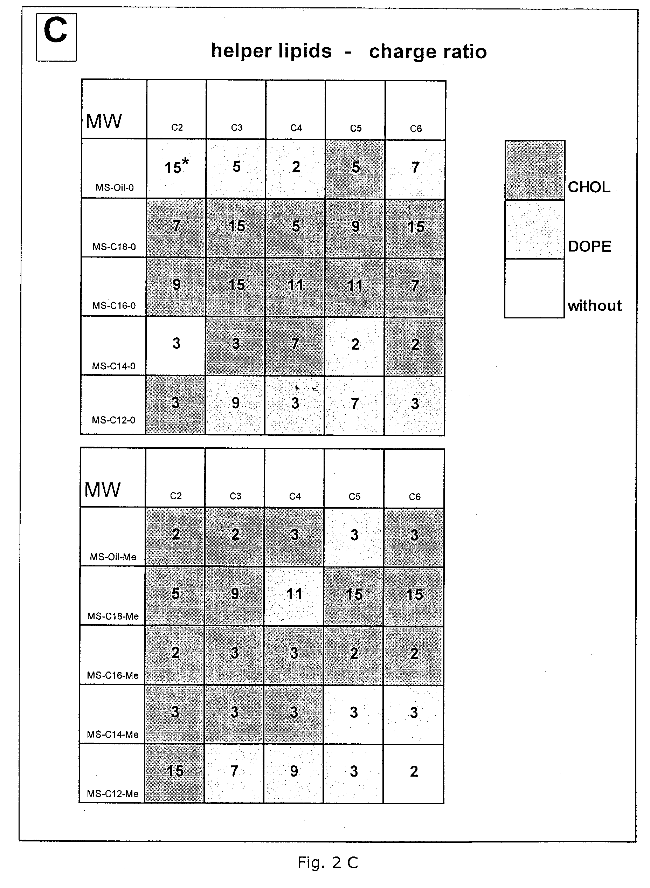

Figure 2 A-C: Efficiency of pGFP-Luc plasmid expression following lipofection with a library of a new class of L-Serin-Scaffold based lipids into COS-7 cells. From each of the fifty MS-lipids (Tab.1 and Fig.1), either non-methylated (A) or methylated (B), twenty four different lipoplexes were formed containing either no helper lipid or the helper lipids DOPE (Dioleoylphosphatidylethanolamine) or CHOL (cholesterol) at molar amounts (1:0.5), respectively. The three lipid mixtures were used at eight different lipid:DNA-charge ratios (LDR) from 1:1 - 15:1 in HEPES-buffer pH 7.4. Transfection was performed in 96-well plates in triplicates as described in the Examples. Results are shown as mean values from two-three independent transfection experiments for each lipid and lipid combination [in the case of lipids MS-11 (MS-C12-0-C4), MS-31 (MS-C12-Me-C3), and MS-46 (MS-C12-Me-C6) only one experiment was performed]. Lipofection efficiencies are shown in RLU (lu µg⁻¹ protein) only for the most effective cationic/helper lipid combination and lipid:DNA ratio for each lipid (A, B). Used cationic/helper lipid combination (shaded boxes) and lipid:DNA charge ratio (internal number^{*}) are shown in (C).

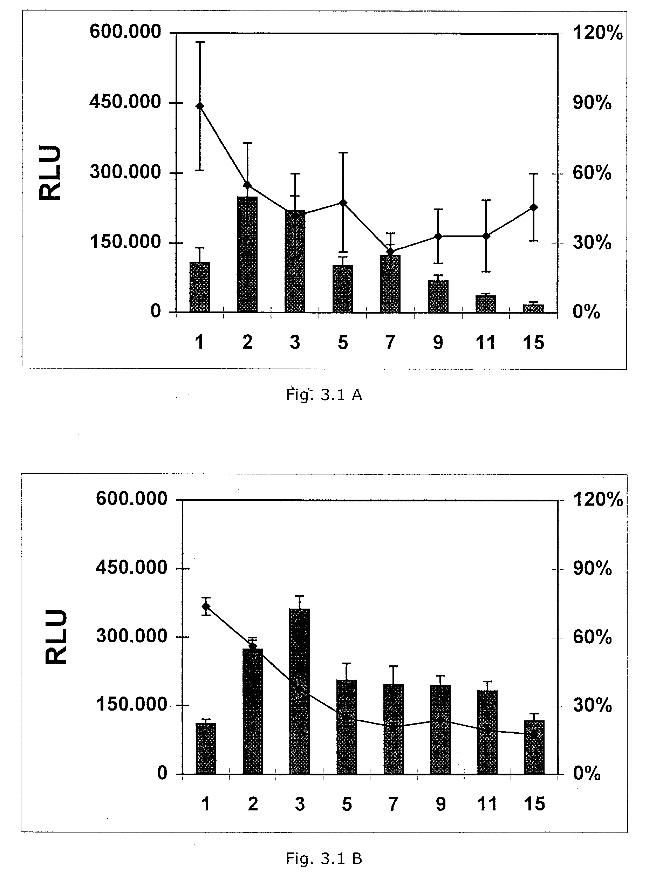

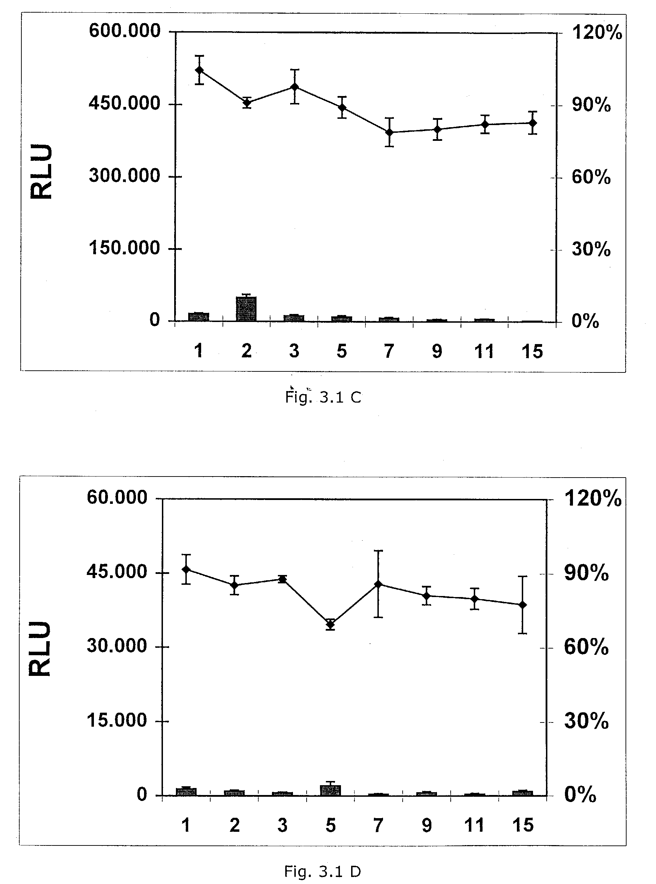

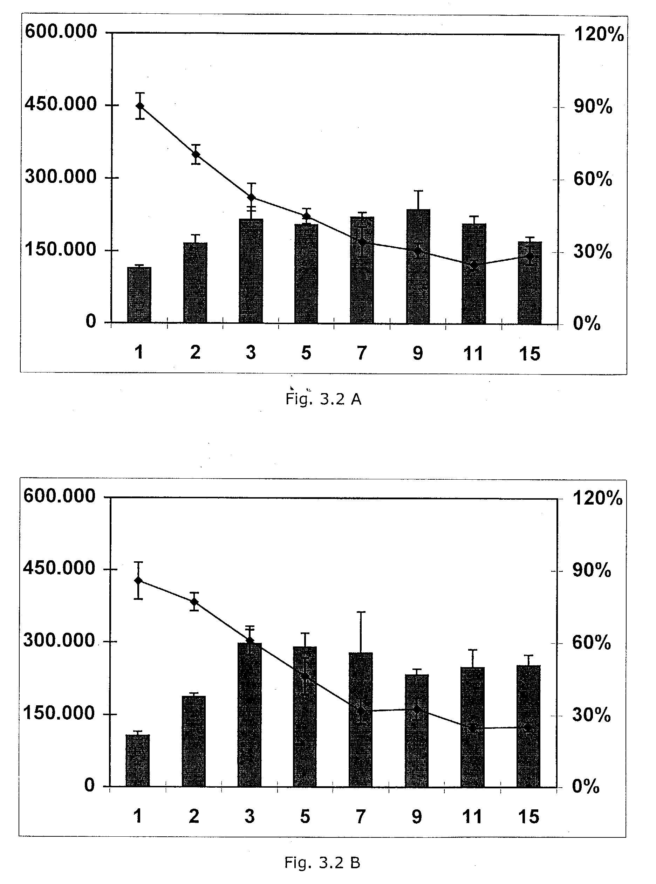

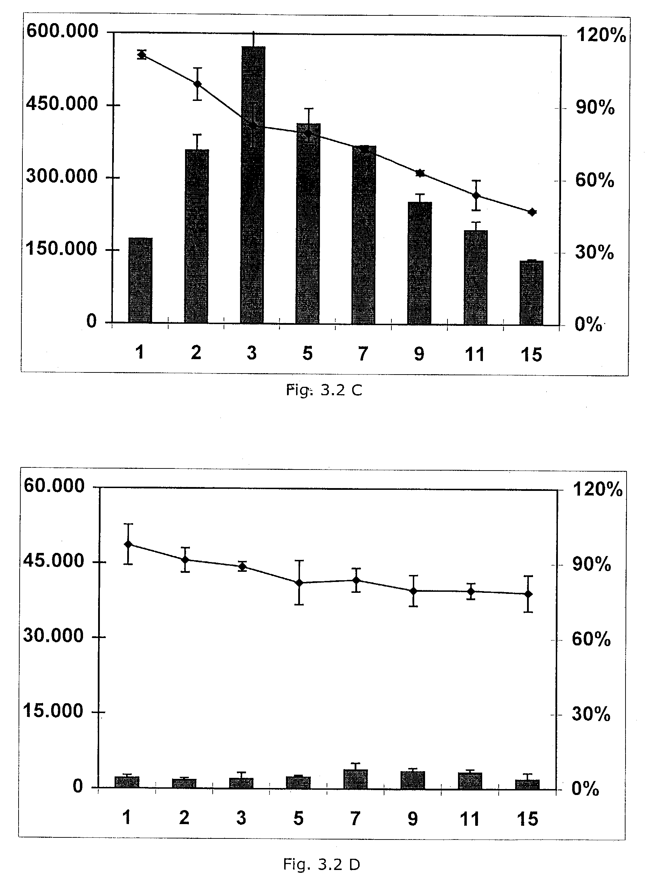

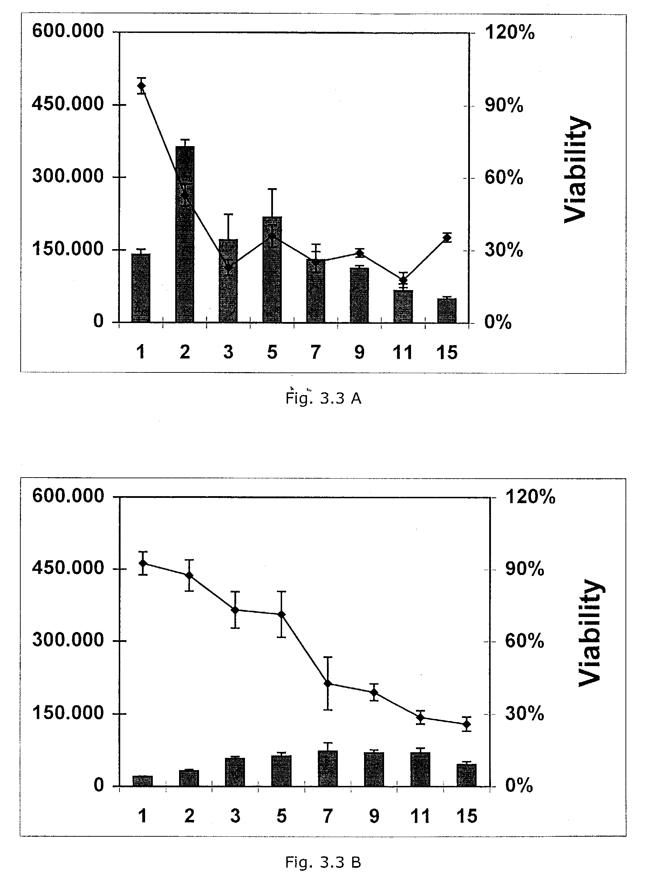

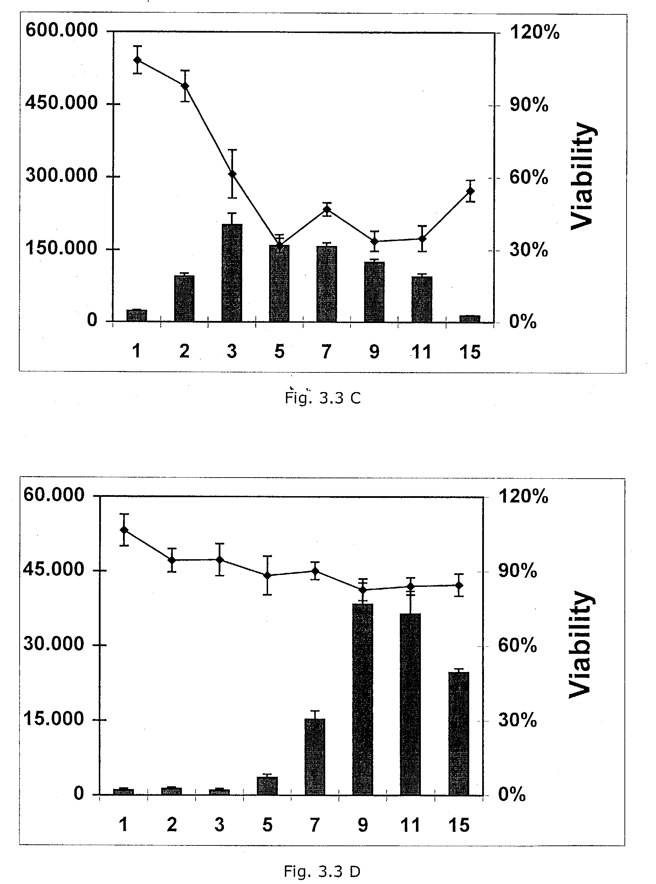

Figure 3.1 A-3.3 D: Transfection diagrams for lipids MS17 (MS-C14-C5-0) (A), MS2 (MS-C14-C2-0) (B), MS33 (MS-C16-C3-Me) (C), and MS5 (MS-oil-C2-0) (D). From each of the MS-lipids twenty four different lipoplexes were formed containing either no helper lipid or the helper lipids DOPE (Dioleoylphosphatidylethanolamine) and CHOL (cholesterol) at molar amounts (1:0.5), respectively, using eight different lipid:DNA-charge ratios from 1:1 - 15:1 in HEPES-buffer pH 7.4. Transfection was performed in 96-well plates in triplicates as described in the Examples. Results are mean values from two-three independent transfection experiments for each lipid and lipid combination. Abbreviations of lipids: "0" means R² is not a substituent i.e. corresponds to an electron lone pair, "Me" means R² is methyl; for further explanations see table 1.
Figure 3.1 A-D: lipid without any helper lipid.
Figure 3.2 A-D: lipid together with CHOL as a helper lipid.
Figure 3.3 A-D: lipid together with DOPE as a helper lipid.
Values for lipofection profiles (column - y-axis on the left) and viability curves (line - y-axis on the right) are given in RLU (lu µg-1 protein) and % viability, respectively, of untreated control cells for respective lipid/helper lipid compositions (± standard deviations). On the x-axis all eight lipid:DNA charge ratios are represented.

### Detailed Description of the Invention

The compound of formula I according to embodiment (1) of the invention is to be construed comprising the compounds represented by any of the following formulae (Ia) to (Ie) 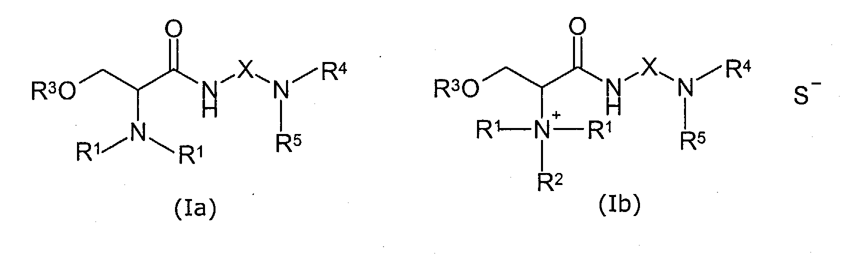 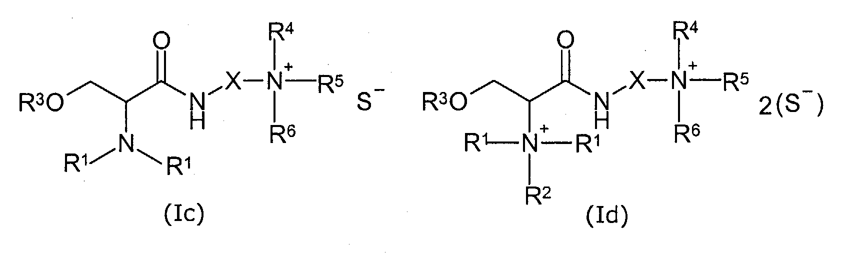 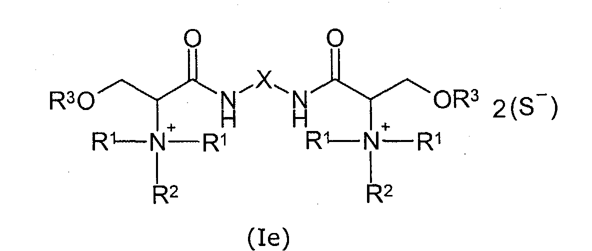 The variables within formulae (Ia) to (Ie) are as defined for formula (I) above. The variables within the formulae (I) and (Ia) to (Ie) (hereinafter shortly referred to as "compounds of the invention") are defined in more detail below.

The alkyl residues R¹ in the compound of the invention are non-polar long chain alkyl residues which can be substituted and/or can be unsaturated (i.e. may have one or more C-C double and/or triple bonds) and can have a linear or branched chain. Said long chain alkyl residues preferably comprise 6 to 26 carbon atoms. In a preferred embodiment R¹ is selected from a linear or branched (chain), preferably linear, C₁₀₋₂₀ alkyl, alkenyl, alkdienyl, alktrienyl and alkinyl residue, more preferably from linear C₁₀, C₁₂, C₁₄, C₁₆, C₁₈ and C₂₀ alkyl and alkenyl residues. In a still more preferred embodiment R¹ is selected from C₁₂, C₁₄, C₁₆, C₁₈ alkyl, and oleyl. In the most preferred embodiment R¹ is selected from C₁₂, C₁₄ and oleyl (in case of compounds of formulae (Ia) and (Ic), which means R² is absent, or compounds of formulae (Ib), (Id) and (Ie) where R² is a hydrogen atom) and is selected from C₁₄, C₁₆ and oleyl (in case of compounds of the formulae (Ib), (Id) and (Ie), where R² is other than hydrogen).
R² and R⁶ in the compounds of the invention are independently selected from a free valency, a hydrogen atom and a lower alkyl residue which can be substituted and/or can be unsaturated (i.e. may have one or more C-C double and/or triple bonds) and can have a linear or branched chain. The lower alkyl residue preferably has 1 to 6 carbon atoms. It is also preferred that R² and R⁶ are the same. In a preferred embodiment of the invention R² and R⁶ are selected from a free valency, a hydrogen atom, and an optionally substituted, linear or branched, preferably linear C₁₋₃ alkyl residue. Such C₁₋₃ alkyl residues include, but are not limited to, methyl, ethyl and hydroxyethyl.

R³ in the compounds of the invention is selected from hydrogen, an alkyl residue, preferably a lower alkyl residue, which can be substituted and/or can be unsaturated (i.e. may have one or more C-C double and/or triple bonds) and/or can contain a heteroatom within its chain; an acyl residue, preferably a lower acyl residue which can be substituted and/or can be unsaturated (i.e. may have one or more C-C double and/or triple bonds), and a suitable (hydroxy) protecting group. Said lower alkyl and lower acyl residue preferably contains 1 to 6 carbon atoms. Said heteroatoms contained in the lower alkyl residue are preferably selected from O, S and N-R⁷ (where R⁷ is a hydrogen atom or an optionally substituted lower alkyl group). In a more preferred embodiment R³ is selected from' hydrogen, a hydrophilic C₁₋₆ alkyl residue which can have hydrophilic substituents, a hydrophilic C₁₋₆ acyl residue, and a polar hydroxyl protecting group such as THP, MEM, MOM, Ac, Tos, etc. The hydrophilic C₁₋₆ alkyl residue includes a polyalkoxy alkyl residue of the formula -[(CH₂)ᵣO]ₛ-R⁷ (where r is 2 or 3, preferably is 2; s is an integer range from 1 to 150, preferably in the range from 1-50; and R⁷ is hydrogen or an optionally substituted lower alkyl group, preferably is hydrogen or -(CH₂)ᵣ₋₁CH₃), and a polyethylene glycol (PEG) residue -(CH₂CH₂O)ₛOH (where s is as defined above) or a functionalized derivative of said PEG residue carrying a moiety capable of binding to proteins and peptides (such as a *p*- or *m*-maleiimidobenzoic ester, etc.). In the most preferred embodiment R³ is hydrogen.

In the compounds of the invention R⁴ and R⁵ are independently selected from a hydrogen atom, an alkyl residue, preferably a lower alkyl residue , which can be substituted and/or unsaturated (i.e. may have one or more C-C double and/or triple bonds) and/or can contain a heteroatom within its chain, an acyl residue, preferably a lower acyl residue, which can be substituted and/or unsaturated (i.e. may have one or more C-C double and/or triple bonds) a suitable (amino) protecting group, and an optionally derivatized amino acid or polypeptide residue including a residue of formula (II) 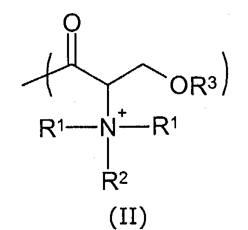 (where all the variables are as defined above). Said lower alkyl and lower acyl residue preferably contains 1 to 6 carbon atoms. Said heteroatoms contained in the lower alkyl residue are preferably selected from O, S and N-R⁷ (where R⁷ is a hydrogen atom or an optionally substituted lower alkyl group. A "derivatized amino acid" or "derivatized polypeptide" according to the invention refers to usual modifications of naturally occurring amino acids and polypeptides derived therefrom including alkylation and acylation of hydroxy, and amino functionalities present therein, introduction of protective groups, etc.

In a more preferred embodiment R⁴ and R⁵ are independently selected from hydrogen, an optionally substituted C₁₋₆ alkyl, alkenyl and alkinyl residue, a C₁₋₆ acyl residue having one or more C-C double and/or triple bonds, an amino protecting group including, but not limited to, carbamate forming groups (such as Boc, Troc, and Bn, Tos, allyl, Fmoc, Bz, etc.), and other amino protecting groups known in the art (e.g. as mentioned in T.W. Green, Protective Groups in Organic Synthesis, Wiley, New York, 2^{nd} Ed. (1991)); the residue of formula (II) above. In the most preferred embodiment R⁴ and R⁵ are hydrogen.

In yet another embodiment R⁴ and R⁵ together with the adjacent nitrogen form an optionally substituted heterocyclic ring. Said heterocycle may contain further heteroatoms such as N, O and S. Preferably the heterocycle is 5- to 7-membered. Particularly preferred heterocycles include, but are not limited to, pyrolidino, imidazolino, piperidino, morpholino, pyridino, azepano, etc.

In the compounds of the invention X represents a linear or branched alkylene residue which can be unsaturated, and/or can be substituted and/or can contain a heteroatom within its chain. Said alkylene residue preferably has 2 to 10 atoms, and said heteroatoms contained within the alkylene chain are preferably selected from O, S and N-R⁷ (where R⁷ is a hydrogen atom or an optionally substituted lower alkyl group). In a preferred embodiment X is linear C₂₋₁₀ alkylene residue, which can be substituted and/or can contain a heteroatom within its chain. In a more preferred embodiment X is an optionally substituted linear C₂₋₁₀ alkylene residue, most an optionally substituted linear C₂₋₆ alkylene residue.

For the most preferred lipid according to the invention R¹ is selected from C₁₂, C₁₄, C₁₆, C₁₈ alkyl, and oleyl, preferably in case R² is a fee valnecy or a hydrogen atom, R¹ is selected from C₁₂, C₁₄ and oleyl and - in case R⁶ is a methyl group, R¹ is selected from C₁₄, C₁₆ and oleyl; R² is a free valency, a hydrogen atom or methyl; R³, R⁴ and R⁵ are hydrogen atoms; and X is an optionally substituted linear chain C₂₋₆ alkylene residue.

As for R¹ it is particularly preferred to utilize naturally occurring C₁₄, C₁₆, C₁₈, C₂₀, C₂₂ fatty acid derivatives, e.g., residues derived from oleic acid, linoleic acid, linolenic acid, cis-11-eicosanoic acid, arachidonic acid and erucic acid, or the aldehydes or alcohols corresponding thereto.

The above lower alkyl groups having about 1 to 6 carbon atoms within the definitions of R², R³, R⁵ and R⁷ include linear and branched, saturated and unsaturated residues such as methyl, ethyl, propyl, isopropyl, n-butyl, sec-butyl, tert-butyl, n-pentyl, n-hexyl, allyl and the like.

The above term "substituted" within the definitions of R¹, R², R³, R⁴, R⁵, R⁶, R⁷, the heterocycle and X means that suitable substituents are present on the alkyl, alkenyl, alkinyl, alkylene, acyl and heterocycle residues. Said substituents include, but are not limited to, the following groups: hydroxy, oxy, halogen (such as fluorine, chlorine, bromine etc.), lower alkyl (a linear or branched alkyl group having about 1 to 6 carbon atoms as defined hereinbefore), lower alkoxy (where the alkyl portion is as defined hereinbefore) such as methyl ethyoxy, propoxy and the like, lower acyl (such as formyl, acetyl, propionyl, butyryl, etc.), acyloxy (including, but not limited to, formyloxy, acetyloxy, propionyloxy, seryloxy, glycyloxy, alanyloxy etc.), mercapto, lower alkyl thio (such as methylthio, ethylthio, propylthio, isopropylthio, butylthio, etc.), mono- and di-lower alkyl amino, and the like. The hydrophilic substituents among the substituents mentioned above are well-known for a person skilled in the art and include, e.g., hydroxy, oxy, methoxy, formyl, acetyl, etc. The number of such substituents on the alkyl residues of R¹, R², R³, R⁴, R⁵, R⁶ R⁷, the heterocycle and X are 1-5, preferably 1-3.

The biologically and/or pharmaceutically acceptable counterion(s) n(R⁻) required to neutralize the positive charge of the compounds of the invention include any mono-, di- or polyvalent counterion which does not adversely affect the transfer of the biologically active molecule into the cell or tissue can be utilized. Preferred counterions include, but are not limited to, carboxylates (such as acetate, propionate, etc.), fluorinated carboxylate (such as monofluoroacetate, difluoroacetate and trifluoroacetate, perfluoropropionate and the like), sulfate, alkylsulfates (such as methylsulfate, ethylsulfate, propylsulfate and the like), fluorinated alkylsulfates (the alkylsulfates mentioned hereinbefore containing at least one fluorine atom), arylsulfates (including alkylaryl sulfates such as benzylsulfate, toluylsulfate and the like), fluorinated arylsulfates (the arylsulfates mentioned hereinbefore containing at least one fluorine atom) and halides (such as chloride and bromide).

Most preferred compounds of embodiment (1) are shown in the following Table 1, which provides a library of cationic lipids with L-serine scaffold with varying length of the non-polar hydrocarbon chains (R¹) and of the side chains (X).

The lipids were synthesised either non-methylated or methylated (R²) at the tertiary amine.

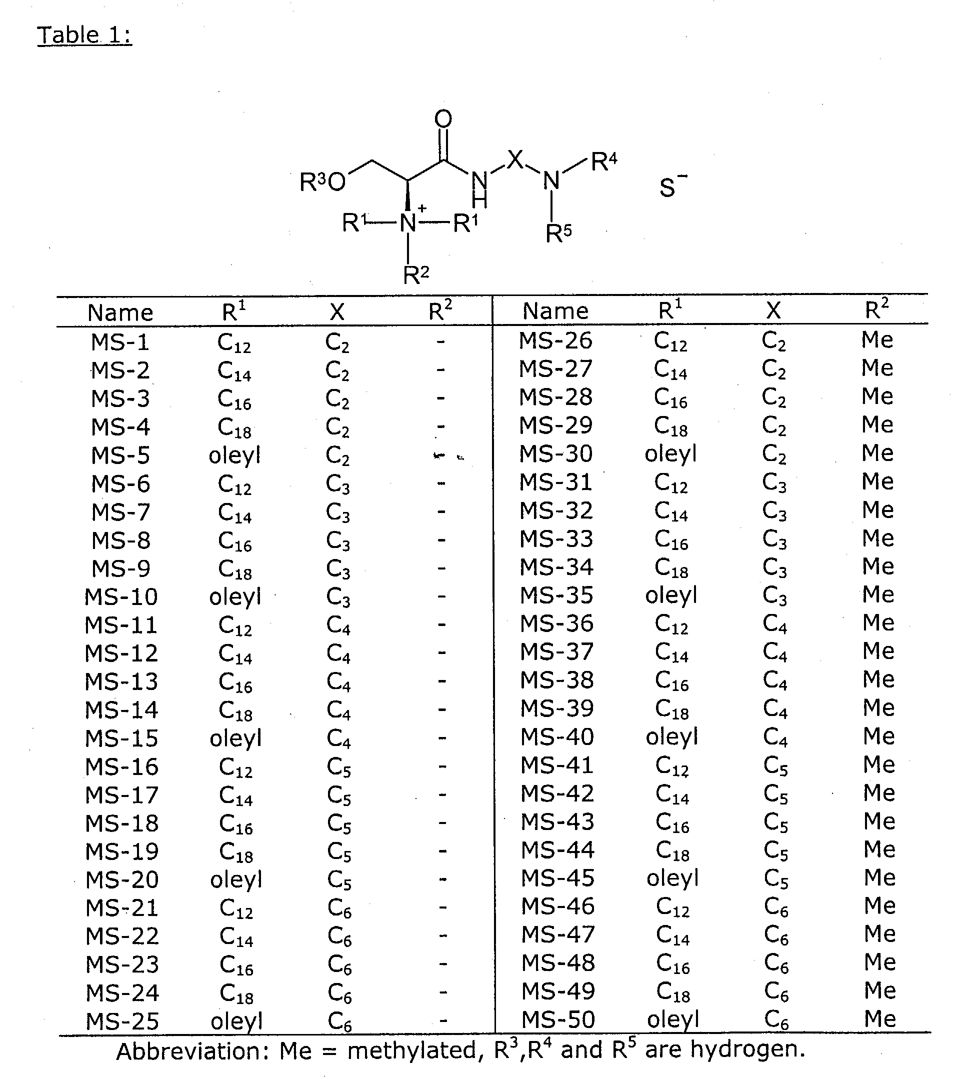

The configuration of C(1) of the serine moiety of the lipid according to formulae (I) and (Ia) to (Ie) of the invention is racemic or has either *R* or *S* configuration (according to the Cahn-Ingold-Prelog (CIP) nomenclature), preferably has S-configuration (i.e. the serine moiety is derived from proteinogenic L-serine), e.g. as depicted in formula (I.2) 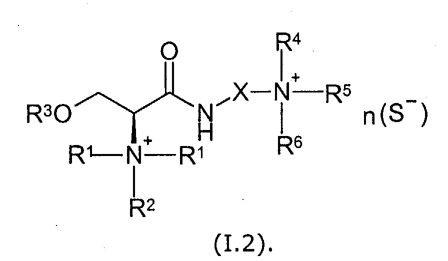 Likewise, the configuration of C(1) of the serine moiety of the residue according to formula (II) of the invention is racemic or has either *R* or *S* configuration (according to the CIP nomenclature), preferably has *S*-configuration (i.e. the serine moiety is derived from proteinogenic L-serine) as depicted in formula (II.2)

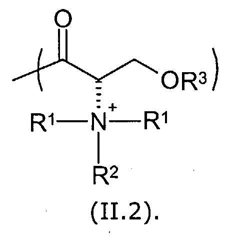

In accordance with embodiment (2) of the invention the biologically active molecules (b) include, but are not limited to, nucleic acid constructs, DNA, RNA, synthetic polynucleotides, antisense polynucleotides, missense polynucleotides, nonsense polynucleotides, ribozymes, proteins, peptides, small molecular weight drugs, antibiotics and hormones.
The composition of embodiment (2) of the present invention may additionally comprise a helper lipid (c) which differs from the cationic lipid of formula (I), or a mixture of such helper lipids. The helper lipid(s) is/are preferably selected from phosphatidylcholine, lyso-phospatidylcholine, phosphatidylethanolamine and lyso-phosphatidylethanolamine and the like (having saturated or unsatured carbon chains of variable length); sphingomyelin, ceramide and the like; membrane forming amphiphiles such as block polymers and the like; alkylester, -ether, -amides of sugars, dioles, polyoles, amines, amino acids, peptides and the like; cationic or lipophilized polymers such as DEAE-dextran, histones, polyethylene imine and the like; cholesterol; phosphatidylglyceroles, phosphatidylserines, phosphatidylinositoles and the like; helper agents modulating uptake of lipoplexes (the complex between cationic lipid (a) and biologically active molecule in the composition of embodiment (2) of the invention is hereinafter shortly referred to as "lipoplex") by macrophages and other cells such as gangliosides, phosphatidylpolyglycideroles, PEG-phosphatidylethanol-amines, proteins (including IgG and Fc-fragments) and the like. Most preferable the helper lipid is dioleoylphospatidylethanolamine (DOPE) or cholesterol.
In accordance with the present invention the molar ratio of (c)/(a) is from 0.01 to 20, preferably 0.1 to 5, and more preferably is from 0.3 to 1.2 in the case of DOPE and from 0.3 to 1.0; yet more preferably from 0.3 to 0.7 in the case of CHOL.
In accordance with the present invention the lipid:DNA charge ratio is 1:1 to 15:1.
In a further preferred embodiment of the invention the composition of embodiment (2) is a composition for gene therapy, i.e., is suitable for transferring a biologically active molecule into mammalian, preferably human cells.
The present invention also provides the use of a cationic lipid of formulae (Ia) to (Ie) as defined herein before as a transfection agent for transferring a biologically active molecule into all types of cells and/or tissues (i.e., in an *ex vivo* application), or for preparing an agent for gene therapy (i.e., *in vivo* application) for mammals including humans. Besides the biologically active molecule and the cationic lipid, the agent may further comprise a helper lipid as defined herein before.
As for the process according to the invention the term "conditions suitable for carrying out a reductive amination" includes all the conditions well known to the synthetic organic chemist who wants to carry out reductive amination. In particular, any reducing agent suitable for reductive amination can be used, such as a hydrogen in the presence of a suitable catalyst, zinc in an acidic medium, sodium cyanoborohydride, sodium triacetoxyborohydride, sodium borohydride, iron pentacarbonyl in the presence of alcoholic KOH, BH₃-pyridine, and formic acid. Preferred are mild reducing agents such as sodium cyanoborohydride and sodium triacetoxyborohydride, most preferred is sodium triacetoxyborohydride.

Synthetic strategy: For the synthetic screening approach described here a large number of lipids had to be synthesised. This was accomplished by a combinatorial solid phase synthesis. The synthetic strategy was based on the utilisation of 4-methoxytrityl chloride resin and, as far as possible, commercially available building blocks. This strategy was used to synthesise the new class of transfection lipids with a L-serine-scaffold presented in this study. The synthetic route is outlined in Fig. 1. First step of the synthesis was the immobilisation of Fmoc-protected L-Serine 2 on the 4-methoxytrityl chloride resin 1. (Frechet, J..M.J., Nyens, L.J., Canadian Journal of Chemistry 54:926-34 (1976)) Coupling of the Boc-protected diamine to the immobilised carboxylic acid was achieved by using the coupling reagent PyBOP and yields the polymer-bound amide 5. After deprotection of the Fmoc-protected amino function of 5 by treatment of the resin with piperidine (Zhu, T, Boons, G.J., Angew. Chem. Int. Ed. Engl. 37:1898-1900 (1998)), two non-polar groups are introduced by reaction with a long-chained aldehyde (hexadecanal) (Taber, D.F. et al., J. Org. Chem. 52:5621-2 (1987)). For this reductive amination sodium triacetoxyborohydride is used as a mild reducing agent (Morphy, J.R. et al., Tetrahedron Lett. 37:3209-12 (1996)). In order to get a constantly cationic lipid, the tertiary amine 8 is methylated with methyl iodide (Brown, A.R. et al., J. Am. Chem. Soc. 119:3288-95 (1997)). Treatment of the resin with 50 % trifluoroacetic acid released the lipid from the solid phase and deprotected the amino group. The lipid 10 was purified by preparative HPLC. This synthetic strategy allowed us the parallel synthesis of a lipid library containing 50 different, structurally near related compounds based on a L-Serine scaffold with varying length (R¹) of the non-polar hydrocarbon (C₁₂, C₁₄, C₁₆, C₁₈, or oleyl-) chains, varying length (X) of head groups (C₂, C₃, C₄, C₅, or C₆) and (R²) non-methylated or methylated at the tertiary amine (Table 1).

Screening/Lipofection studies: For a broad and reproducible testing of the transfection properties of systematically varied new cationic lipids, we recently have developed a fully automated high throughput system (HTS). This system, which comprises the entire lipofection process from liposome formation to reporter gene assay, allows to identify candidate cationic lipids and to develop suitable lipofection reagents and protocols based on those lipids. Automation of this process was necessary, because manually testing of various cationic lipids or lipid mixtures is extremely labor intensive and high reproducibility can not be guaranteed.

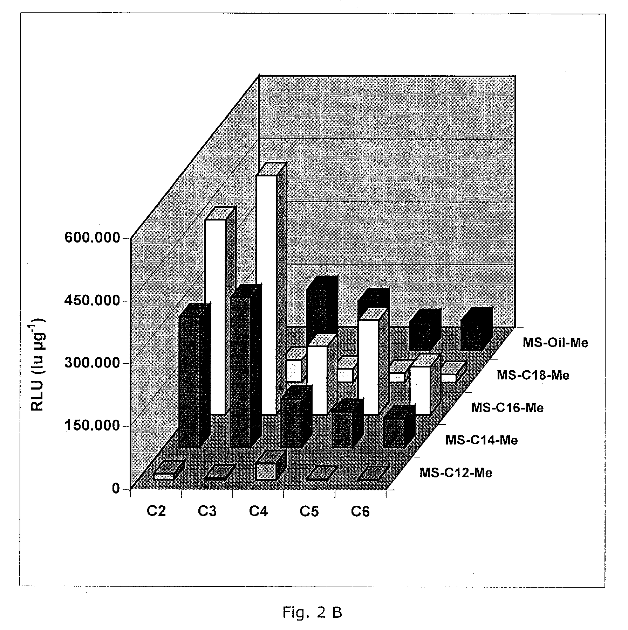

Screening the combinatorial lipid library for lipofection properties: Goal of this first step was to compare transfection efficiency of these structurally related cationic lipids with systematically modified hydrocarbon chain compositions for the development of suitable lipofection reagents and protocols and to study the relationship between structure and transfection efficiency of this new class of cationic lipids for gene transfer. All lipids (MS-1 to MS-50, table 1) were tested using a standard protocol: From each lipid, twenty four different lipoplexes were formed containing either no helper lipid or the helper lipids DOPE and CHOL at molar amounts (1:0.5), respectively, using eight different lipid:DNA-charge ratios (LDR) from 1:1 - 15:1, resulting in a lipofection profile for each lipid. The COS-7 cell-line was used for this lipofection study since COS-7 is easy to transfect and differences between the lipids are easy to identify. For comparison, the transfection efficiency of the well known cationic lipid DOTAP at its best lipid/DNA charge ratio of 2.5 was also determined in each run. Figure 2 shows the most efficiently transfecting combination of helper lipid and LDR (C) of each of the 50 lipids either non-methylated (A) or methylated (B) at the tertiary amine.
Although structurally related the new lipids showed wide variations of transfection efficiency ranging from about 956 to 572.000 RLU (lu µg⁻¹ protein), which corresponds to 0.03 - 18.6 times of the DOTAP values (30.838 ± 6.363 RLU). We could not detect a direct relation between transfection efficiency of individual lipids, the use of helper lipids or optimum LDR (Fig. 2, C). Most lipids of the MS-series displayed highest transfection efficiency in combination with helper lipids CHOL (31/50) or DOPE (16/50) but some of it (3/50) were most active also without adding helper lipids (Fig. 2, C). Helper lipid CHOL showed a slight preference (15/20) in cases with transfection efficiencies >100.000 RLU. Several lipids (e.g. MS-1, MS-12 or MS-17) displayed similar activities in all "helper" lipid combinations (without, CHOL, DOPE), whereas others (e.g. MS-28, MS-29 or MS-33) were most active (2.5 - 11.5 times higher) only in one of the combinations. Optimum LDRs varied non-systematically between 2:1 up to 15:1, however, only from 2:1 - 5:1 in lipids with transfection efficiencies >100.000 RLU (Fig. 2, C).
Most remarkable result of this part of HTScreening, however, was the finding of a dependence of transfection efficiencies on the hydrocarbon chain length of the various MS-lipids and the striking differences between non-methylated (Fig. 2 A) or methylated (Fig. 2 B) but otherwise identical lipids (Table 1). Methylation of the tertiary amine of MS-lipids led to a shift in hydrocarbon chain length of most effective lipids. Whereas in the group of non-methylated MS-lipids, all lipids bearing two C-14 hydrocarbon chains, two lipids with C-12 and one lipid with oleyl hydrocarbon chains (C4) were the most effective, and lipids with C-16 or C-18 hydrocarbon chains only displayed marginal transfection efficiencies (Fig. 2 A), in the methylated group two lipids with C-16 hydrocarbon chain were the most effective lipids in all (Fig. 2 B). In this group the other C-16, two of C-14 and one oleyl hydrocarbon chain-carrying (C2) lipids also displayed significant transfection activity, whereas methylated C-12 hydrocarbon chain-carrying lipids did clearly lack transfection efficiency. C-18 lipids were rather inactive in both groups (Fig. 2 B). Also the C₂ - C₆ X-head groups (Tab. I) did considerably influence transfection efficiency of the lipids (Fig. 2 A: C-12, oleyl; Fig. 2 B: C-16, C-14, oleyl). Comparing methylated and non-methylated MS-series lipids by HPTLC we could clearly demonstrate a different polarity of the two groups of lipids (not shown).

### Examples

Abbreviations: Boc: tert-butoxycarbonyl, (Boc)₂O: di-tert-butyl pyrocarbonate, DHB: dihydroxybenzoic acid, DIEA: *N,N*-diisopropylethylamine, DMF: *N,N*-dimethylformamide, Fmoc: 9-fluorenylmethoxycarbonyl, MALDI-TOF: matrix assisted laser desorption ionization time of flight, PyBOP: benzotriazol-1-yl-oxytripyrrolidinophosphonium hexafluorophophate, TFA: trifluoroacetic acid, THF: tetrahydrofuran, RLU: relative luciferase units (lu µg⁻¹ protein), Rh-DHPE: Lissamine™ rhodamine B 1,2-dihexadecanoyl-sn-glycero-3-phosphoethanolamine, triethylammonium salt, GFP: green fluorescent protein, GFPLuc: green fluorescent - luciferase fusion protein, Me: methylated, LDR: lipid:drug ratio (meist ist aber von lipid:DNA charge ratio die Rede - Konsistenz bitte durchgängig überprüfen), CHOL: cholesterol, DOPE: Dioleoylphosphatidylethanolamine, DOTAP: N-[1-(2,3-dioleoyloxy)propyl]-N,N,N-trimethylammonium methylsulfate, FCS: fetal calf serum, HPTL: high performance thin-layer chromatography, QBI: Quotients of mean fluorescence of internalization and binding; the numbering of the compounds synthesised in the examples refers to the corresponding numbers and compounds as depicted in Fig. 1.

### Materials and Methods

Chemicals and Reagents: Tetrahydrofuran, dichloromethane, dioxane and *N,N*-dimethylformamide were purchased from J. T. Baker. *L*-Serine, *N,N*-diisopropylethylamine, di-tert-butylpyrocarbonate, piperidine, dodecanal, tetradecanal, sodium triacetoxyborohydride and methyl iodide were from Aldrich. PyBOP and 4-methoxytrityl chloride resin were from Nova Biochem. Tetrahydrofuran was distilled over sodium prior to use, dichloromethane and *N,N*-dimethylformamide were distilled over CaH₂.
Equipment: Preparative HPLC was carried out on a Shimadzu LC-8A chromatograph equipped with an evaporative light scattering detector (Polymer Labs). Preparative YMC-PVA-columns were used at a flow rate of 15 ml/min. All lipids were purified using CHCl₃/MeOH (1:1).
MALDI-TOF-spectra were collected using a Perseptive Voyager DE-RP spectrometer (2,5-dihydroxybenzoic acid (DHB) as matrix).
¹H-NMR-spectra were recorded on a Bruker DRX-400 spectrometer.

Synthesis of Fmoc-protected L-serine 2: To a cooled solution of *L*-Serine (5.00 g, 47.6 mmol) and sodium carbonate (6.56 g, 62 mmol) in 75 ml water and 25 ml acetone was added a solution of Fmoc-Cl (14.65 g, 56.6 mmol) in 100 ml acetone. After complete addition, stirring was continued for 4 h at room temperature. The mixture was poured into 200 ml of water and extracted with dichloromethane. The aqueous phase was acidified with HCl and extracted 3 times with dichloromethane. The Fmoc-Serine 2 (11.1 g,) obtained after evaporation of the combined organic layers was used directly for the preparation of 3. ¹H-NMR (400 MHz, CDCl₃): δ (ppm) = 3.89 (d, 1H, J = 11.1 Hz, β-CH*H*); 3.98 (d, 1H, J = 11.1 Hz, β-CH*H*, 4.21-4.40 (m, 4H, α-C*H*, CHC*H*₂O); 6.20 (d, 1H, J = 8.0 Hz, N*H*); 7.28-7.86 (m, 8H, Fmoc-Ar-C*H*).

### Synthesis of Boc-protected diamines

Synthesis of *tert*-butyl-*N*-(4-aminobutyl)-carbamate 4: To a cooled solution of diaminobutane (40 g, 0.45 mol) in 200 ml dioxane was added a solution of (Boc)₂O (12.25 g, 56 mmol) in 200ml dioxane. After addition, the reaction mixture was allowed to warm to room temperature and stirred for 24 h. After the solvent was evaporated, the oily residue was dissolved in ethyl acetate and extracted several times with saturated NaCl-solution. The solvent was removed under reduced pressure to yield 7.4 g of a colourless liquid which was used directly for the synthesis of 5. ¹H-NMR (200 MHz, CDCl₃): δ (ppm) = 1.22 (s, 2H, N*H*₂); 1.44 (s, 9H, C(C*H*₃)₃); 1.36-1.60 (m, 4H, CH₂(C*H*₂)2CH₂); 2.74 (t, J = 6.5 Hz, 2H, C*H*₂NH₂); 3.13 (m, 2H, C*H*₂NHBoc); 4.70 (s, br, 1H, N*H*Boc).
*tert*-Butyl-*N*-(4-aminoethyl)-carbamate, *tert*-butyl-*N*-(4-aminopropyl)-carbamate, *tert*-butyl-*N*-(4-aminopentyl)-carbamate, *tert*-butyl-*N*-(4-aminohexyl)-carbamate were prepared in a similar fashion to 4.

Synthesis of hexadecanal 7: To a cooled solution of hexadecanol (10.0 g, 41 mmol) in dried dichloromethane was added dimethyl sulfoxide (6.43 g, 82 mmol) and P₂O₅ (11.7 g, 82 mmol). After stirring 45 min at room temperature the mixture was cooled to 0 °C and triethylamine (14.52g, 143.5 mmol) was added. The reaction mixture was allowed to warm to room temperature and stirred for 2 h. After addition of 50 ml HCl (10 %) the reaction mixture was extracted 3 times with dichloromethane. The combined organic layers were extracted with saturated NaCl-solution and dried with NaSO₄. After evaporation a colourless solid (6.3 g) was obtained which was purified by column chromatography over silica gel with cyclohexane/ethyl acetate (4:1). ¹H-NMR (200 MHz, CDCl₃): δ (ppm) = 0.9 (t, 3H, J = 6.6 Hz, C*H*₃); 1.3 - 1.5 (b, 26 H, -CH₂-); 2.4 (td, 2H, C*H*₂CHO); 9.7 (t, 1H, CH₂C*H*O).

Octadecanal and oleylaldehyde were synthesised in a similar fashion.

Lipid-synthesis: Lipids were synthesised using 4-methoxytrityl chloride resin. All reactions were carried out in dried solvents and under Ar.

Solid phase synthesis of acid 3: A solution of Fmoc-serine (283 mg, 0.9 mmol) and *N,N*-diisopropylethylamine (135 mg, 1.05 mmol) in anhydrous tetrahydrofuran was added to 4-methoxytrityl chloride resin (50 mg, 0.09 mmol). After stirring the suspension for 48 h, the resin was filtered off and washed several times with CH₂Cl₂/methanol/*N,N*-diisopropylethylamine (17:2:1), THF and CH₂Cl₂.

Solid phase synthesis of amide 5: A solution of *tert*-butyl-*N*-(4-aminobutyl)-carbamate 4 (170 mg, 0.9 mmol) and *N,N*-diisopropylethylamine (230 mg, 1.8 mmol) in dichloromethane was stirred 10 min at room temperature and added to a suspension of resin 3 in a solution of PyBOP (460 mg, 0.9 mmol) in dichloromethane. Stirring is continued at room temperature. After 48 h the resin was filtered off and washed with THF and CH₂Cl₂.

Solid phase synthesis of 6: Removal of the Fmoc-group was achieved by stirring the resin 6 in piperidine/dichloromethane (1:1) for 2 h at room temperature. After this the resin was filtered off and washed with methanol and CH₂Cl₂.

Solid Phase Synthesis of 8: Resin 6 and sodium triacetoxyborohydride (184 mg, 0.9 mmol) were suspended in anhydrous dichloromethane. Hexadecanal 7 (216 mg, 0.9 mmol) was added and the reaction mixture was stirred for 3 h. The resin was filtered off and washed with methanol and CH₂Cl₂.

Solid Phase Synthesis of 9: To resin 8 was added a solution of methyl iodide (1.22g, 9 mmol) in *N,N*-dimethylformamide. After 48 h of stirring at room temperature the resin was filtered off and washed with N,N-dimethylformamide and CH₂Cl₂.

Cleavage, deprotection and isolation of the lipids: After the final synthetic step the resin was treated with a 50 % solution of trifluoroacetic acid in dichloromethane, which led to the cleavage of the lipid 10 from the resin and also the deprotection of the Boc-protected amino function. The resin was filtered off and washed several times with CHCl₃/MeOH (4:1). The combined solutions were evaporated under reduced pressure to yield 31 mg of the crude lipid 10 as a yellowish solid, which was purified by preparative HPLC. ¹H-NMR (400 MHz, CDCl₃): δ (ppm) = 0.8 (t, 6H, C*H*₃); 1.18 (m, 52H, CH₃(C*H*₂)₁₃); 1.58 (m, 4H, NCH₂C*H*₂); 2.89 (m, 4H, NC*H*₂); 3.11 (m, 2H, C*H*₂CH₂NH); 3.14 (m, 2H, NCH₂); 3.2 (s, 3H, NC*H*₃); 3.41 (m, 2H, C*H*₂CH₂NH); 3.99 (m, 2H, C*H*₂OH); 4.13 (m, 1H, C*H*CH₂OH); 7.96 (b, 2H, N*H*₂); 8.70 (b, 1H, N*H*CO). MS (MALDI-TOF, DHB): m/z = 639.84 [M⁺].
The lipids synthesized for the present study are listed in Table 1. All lipids were prepared in a similar fashion to the procedure described above and were identified by MALDI-TOF. For the synthesis of this lipid library Boc-protected diaminoethane, diaminopropane, diaminobutane, diaminopentane, diaminohexane and the long chained aldehydes dodecanal, tetradecanal, hexadecanal, octadecanal and oleylaldehyde were used. Synthetic strategy has been schematically summarized in Figure 1.

High performance thin-layer chromatography (HPTLC): HTPLC plates consisted of silicagel 60, 100 x 200 mm (Merck, Darmstadt, Germany). For chromatography, the plates were prerun with the mobile phase CHCl₃, CH₃OH, H₂O, ammonium hydroxide solution (65:25:4:0.4, v/v). Lipid dispersions were diluted in CHCl₃, CH₃OH (2:1, v/v) to a final lipid concentration of 5.16 mM. Ten microlitres of samples of lipid pairs either non-methylated or methylated (Tab.1) were streaked automatically to the silicagel plates with a Linomat IV (Camag, Berlin, Germany). After evaporation of the solvent the plates were developed in glass tanks with the mobile phase over a distance of 9.0 cm. After development, the plates were dried at 180 °C on heating plate (Thermoplate S, Desaga, Heidelberg, Germany) for 10 min. In order to visualize the lipids by staining the HPTLC plates were dipped automatically 3 times for 2 s in a copper sulfate dye solution (14.7 %, w/v) in phosphoric acid (10 %, w/v) by Chromatogram Immersion Device III (Camag, Berlin, Germany) and dried on the heating plate at 160°C for 6 min [Ziroli, V., unpublished data].

### Transfection studies

Cell culture: SV40 transformed African green monkey kidney cell line COS-7 (ATCC CRL-1651) was routinely passaged in DMEM (GIBCO BRL, Karlsruhe, Germany), respectively, supplemented with 1% L-glutamin solution (100x), 1% Penicillin/Streptomycin solution (100x) (GIBCO BRL, Karlsruhe, Germany), and 10% FCS (BioWhittaker, Verviers, Belgium) at 37°C with 5% CO₂ in humidified atmosphere. For the screening cells were maintained in 96-well culture plates (Greiner, Frickenhausen, Germany) in 100 µl DMEM, 10 % FCS, 1 % Penicillin-streptomycin in a humidified atmosphere at 37 °C/5 % CO₂. 24 h prior to transfection, the cells were seeded into the microtiter plate at 10,000 cells per well to reach a confluency of 50 % at the time of transfection.
Dendritic cells: Dendritic cells (DC) were derived from highly purified circulating CD14(+) monocytes: In a first step, peripheral blood mononuclear leukocytes (PBL) from healthy control persons were isolated by Ficoll-Paque (Pharmacia Biotech AB, Uppsala, Sweden) density centrifugation. The following immunomagnetic purification of the monocytes from PBL was performed using CD14 Microbeads and MiniMACS high gradient magnetic separation columns (Miltenyi Biotech, Bergisch Gladbach, Germany) according to manufacturer's instructions. Isolated monocytes (5 x 10⁵/well) were cultured in RPMI supplemented with 10 % FCS, or serum-free Macrophage-SFM medium (GIBCO BRL, Karlsruhe, Germany) supplemented with human recombinant GM-CSF (0.05µg/ml) and IL-4 (0.025µg/ml) (PromoCell, Heidelberg, Germany) in 6-well plates. Aliquots of the cells were reanalyzed by FACS with anti-CD14 mAbs (Pharmingen, San Diego, CA). After 5-6 days non-adherent cells were collected and purity of DCs was determined by FACS analysis using a FACSort flow cytometer (Becton Dickinson, Mountain View, CA) and the CellQuest software for data processing as recently described (Jantscheff, P. et al., J. Immunol. Methods 163(1):91-7 (1993)). For this FACS analysis, HLA class I (clone W6/32), HLA-DR (clone HB55) (kindly provided by Dr. G.DeLibero, Basel, Switzerland), CD1a, CD3, CD11c, CD14, CD54, CD80, CD83 and CD86 specific mAbs (Pharmingen, San Diego, CA) and R-PE-goat anti-mouse Ig (Southern Biotechnology Associates, Inc., Birmingham, AL) were used. Binding specificity was determined using IgG1 (MOPC-21), IgG2a (UPC-10), or IgG2b (MOPC-141) isotype control mAbs (SIGMA-ALDRICH Chemie, Buchs, Switzerland).
Transfection Vector: Vector pEGFPLuc (BD Biosciences Clontech, Palo Alto, CA) is a commercial available reporter vector encoding a fusion protein of green fluorescent protein (GFP) and luciferase (Luc). All informations are available at Clontech homepage
(http://www.clontech.com/techinfo/vectors/vectors E/pEGFPLuc.shtml).

Determination of the transfection properties: Determination of transfection behavior of the new lipids were performed using an automated robot assay system as described in detail by Lenssen et al. and Regelin et al. (Regelin, A.E. et al., J. Biomol. Screen 6(4):245-54 (2001); Lenssen, K. et al., Chembiochem. 3(9):852-8 (2002)). In brief, the method consists of two parts,
(i) the preparation of liposomes and formation of lipoplexes from the different lipids and (ii) cell transfection and determination of transfection properties.
   Pretransfection assays: Cationic lipids in organic solvents were transferred into glass test tubes. The organic solvents were removed under a stream of nitrogen to create a thin lipid film on the surface of the glass tube. HEPES buffer (20 mM plus 130 mM NaCl, pH 7.4) was added and the tubes were transported into a water bath sonicator where a dispersion of small cationic liposomes is formed (1,29 mM). The liposomal dispersions were prepared from DOTAP (N-[1-(2,3-dioleoyloxy)propyl]-N,N,N-trimethylammonium methylsulfate), or from fifty lipids, either non-methylated or methylated, (Tab. 1) of a new class of cationic lipids based on a L-serine scaffold (MS-Series), either without or mixed with the helper lipids (at cationic/helper lipid ratio 1:0.5), DOPE (Dioleoylphosphatidylethanolamine) and CHOL (cholesterol), respectively. Liposomes were distributed to the wells of a 96 deep-well-plate and reporter plasmid DNA was added to allow formation (1 h) of the lipid/DNA-complexes (lipoplexes) at eight different lipid:DNA-charge ratios from 1:1 - 15:1 in HEPES-buffer pH 7.4. For the subsequent quantification of transfection efficiency (part 2) a reporter plasmid carrying the GFP-firefly luciferase gene under the control of the CMV promoter was used to form lipoplexes.
   Lipoplexes from FuGene 6 (Roche Diagnostics, Mannheim, Germany) were formed with the same reporter plasmid DNA, according to manufacturer's instructions.

Transfection assays: After lipoplex formation, cell culture microtiter plates were taken from the incubator, the lid was removed from the plates and 90 µl of the lipoplex dispersions were added to the cells (triplicates) by the robot. FCS concentration during transfection of COS-7 cells was therefore 5.3 %. DC were transfected either at 5.3 % FCS or in serum-free Macrophage-SFM medium. The lids were replaced and the MTP were returned to the incubator. After 14 hours the adherent cells were automatically retrieved, the cell monolayers were carefully washed using the special drop mode of a plate washer, fresh medium was added and the cells were incubated for a further 34 hours before harvesting. The MTP containing the cells were transported to the robot, the medium was removed, and the cells were washed using the drip mode of the microplate washer and lysed. Non-adherent DC were manually washed by centrifugation two times with PBS and lysed. Cell lysates were diluted and aliquots transferred to white microplates for the luciferase activity assay and transparent plates for the BCA protein assay. Assay specific standards and controls were added to the microplates, and luciferase activity and protein content of the lysates were measured. Transfection efficiencies were calculated by dividing luciferase activity by protein content in RLU (lu µg-1 protein). Dividing protein content of transfected cells by protein content of non-transfected control cells resulted in a relative measure of cytotoxicity (% viability). Lipoplexes were formed from DOTAP, FuGene 6 (Roche Diagnostics, Mannheim, Germany), or MS-series lipids, mixed with optimal helper lipids (cationic/helper lipid ratio 1:0.5), DOPE (Dioleoylphosphatidylethanolamine) or cholesterol (CHOL), at eight different lipid:DNA-charge ratios from 1:1 - 15:1 in HEPES-buffer pH 7.4. Transfection was performed in 96-well plates in triplicates as described above. The results are summarised in the following tables 2 and 3 and Fig. 3.

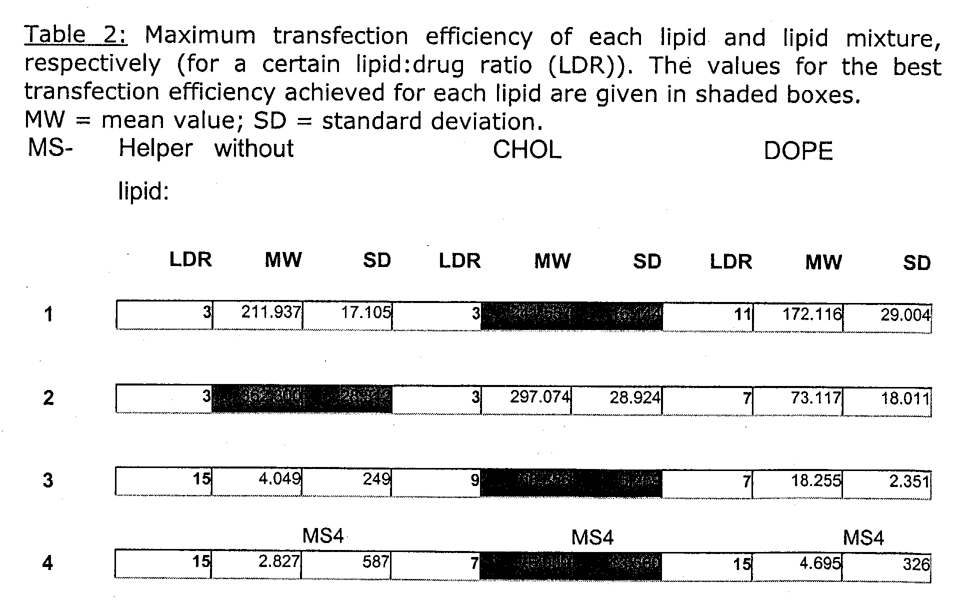

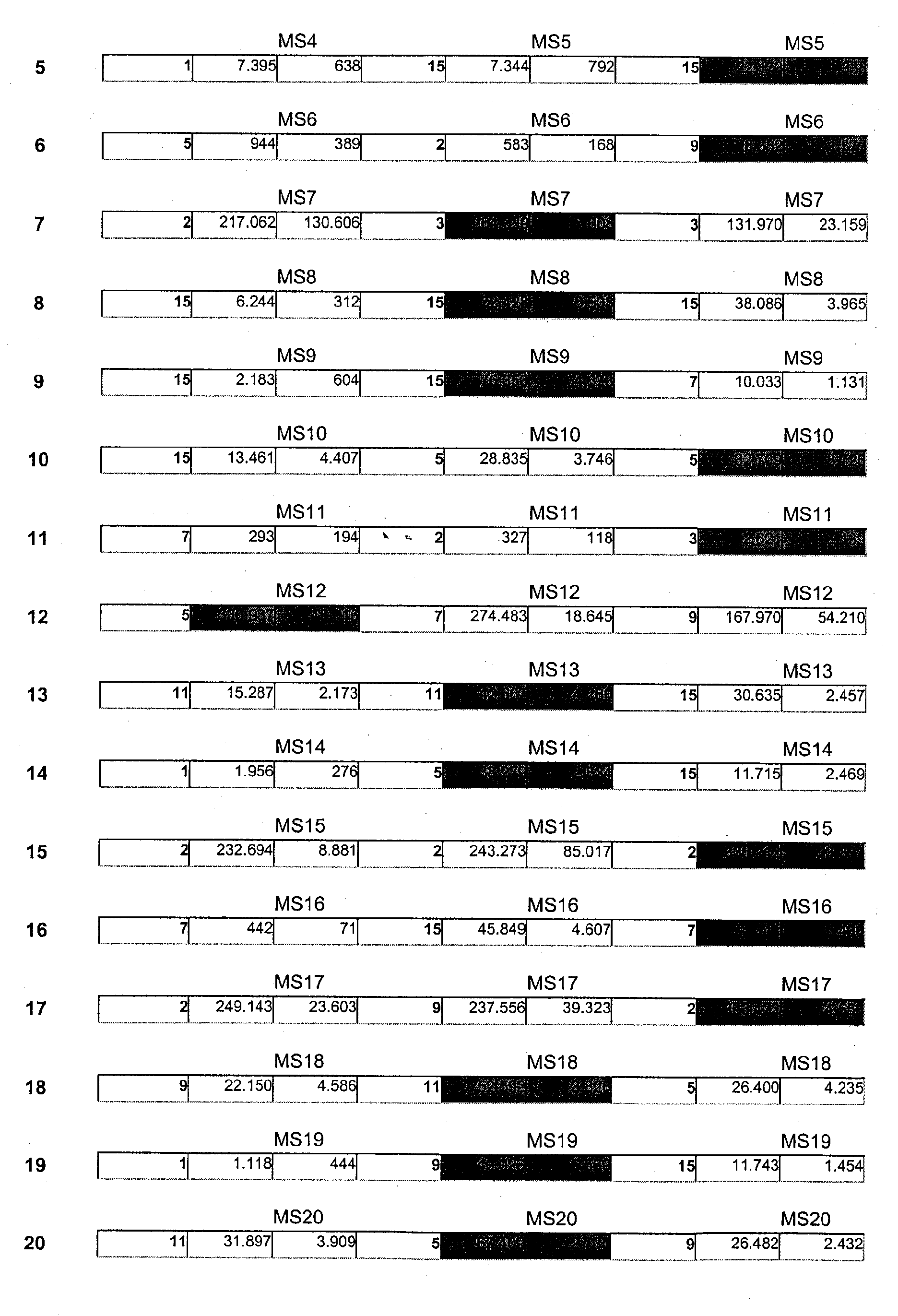

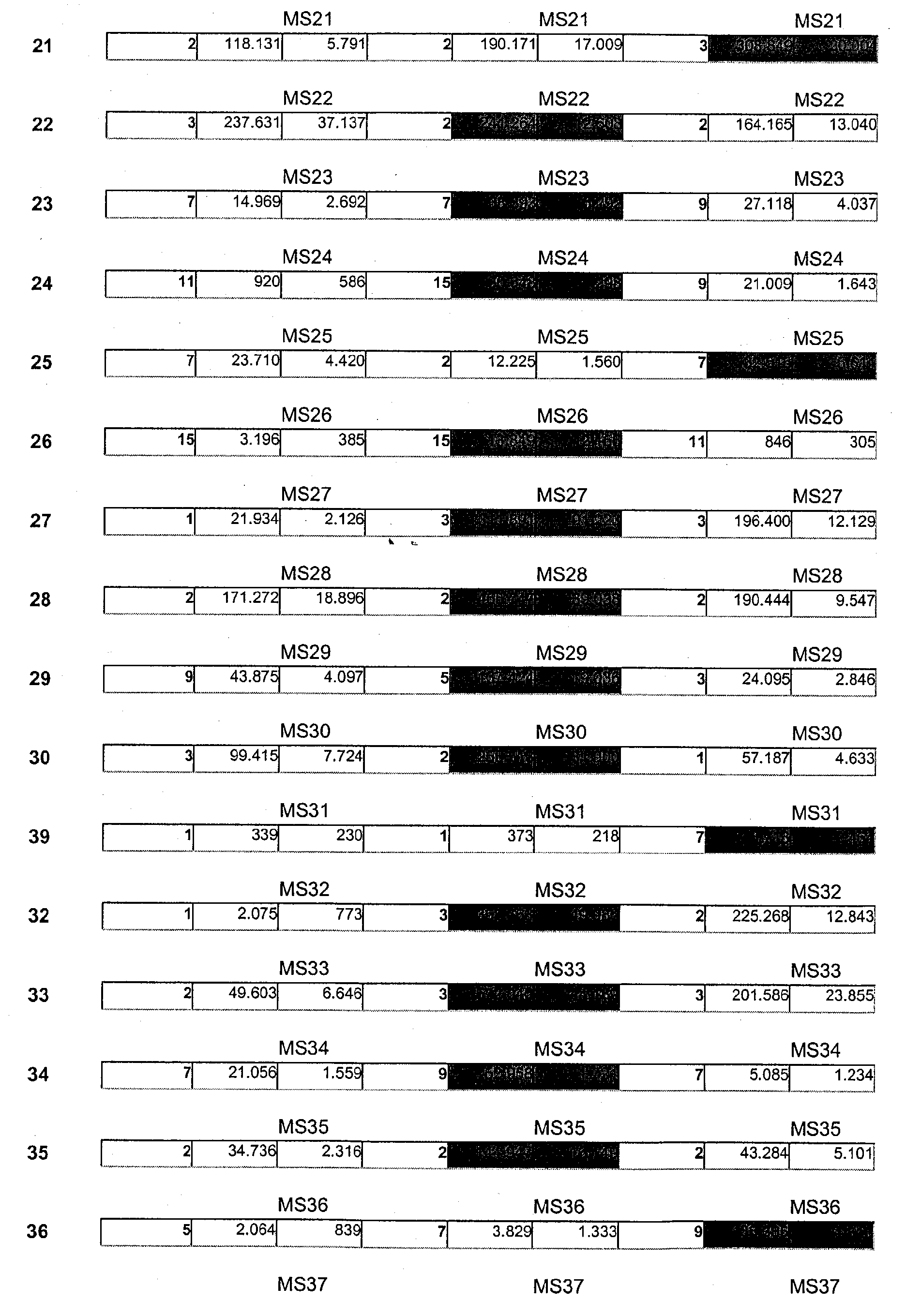

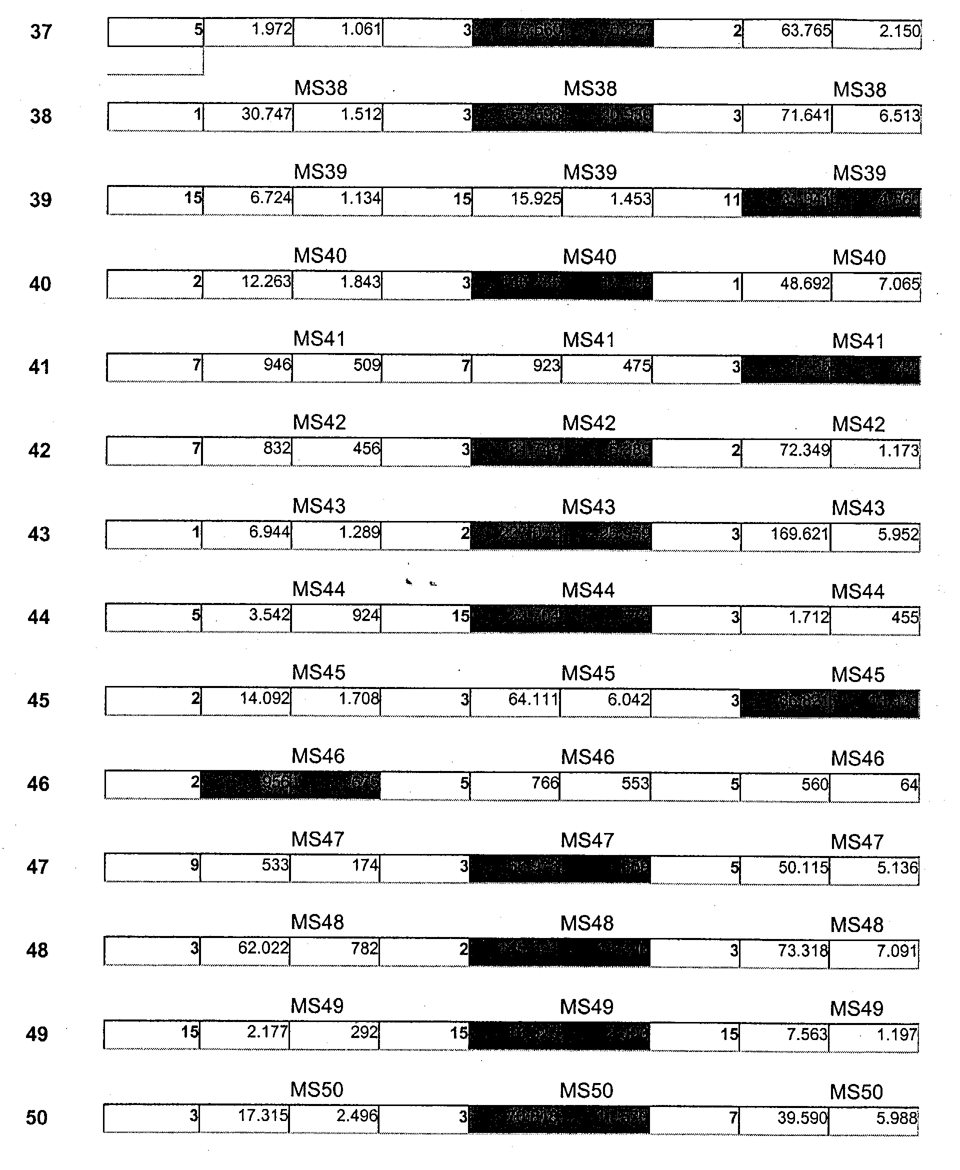

**Table 3:**

| Transfection efficiencies of various MS-lipoplexes in primary dendritic cells | | | |
|---|---|---|---|
| Name | RLU | (n) | % DOTAP |
| MS-21/DOPE | 565 ± 5 | (4) | 240 ± 2 % |
| MS-15/DOPE | 561 ± 66 | (3) | 238 ± 28 % |
| MS-7/CHOL | 497 ± 7 | (4) | 211 ± 3 % |
| MS-6/DOPE | 474 ± 28 | (2) | 202 ± 12 % |
| MS-12/CHOL | 469 ± 25 | (3) | 199 ± 11 % |
| MS-32/CHOL | 429 ± 10 | (3) | 182 ± 4 % |
| MS-17/DOPE | 418 ± 5 | (4) | 178 ± 2 % |
| MS-27/CHOL | 378 ± 3 | (4) | 161 ± 1 % |
| MS-13/CHOL | 377 ± 42 | (4) | 160 ± 18 % |
| MS-8/CHOL | 351 ± 40 | (3) | 149 ± 17 % |
| MS-2/CHOL | 343 ± 64 | (4) | 146 ± 27 % |
| MS-33/CHOL | 343 ± 99 | (4) | 146 ± 42 % |
| MS-28/CHOL | 310 ± 46 | (2) | 132 ± 19 % |
| FuGene 6 | 267 ± 28 | (2) | 113 ± 12 % |
| DOTAP | 235 ± 119 | (13) | 100 % |
| MS-lipids were mixed at molar ratios (1:0.5) with helper lipids (DOPE or CHOL) at optimum lipid:DNA ratios (Fig. 2 C). Transfection efficiencies of the MS-lipoplexes were compared to the transfection efficiency achieved with FuGene 6 (Roche Diagnostics, Mannheim, Germany) and the standard transfection lipid DOTAP. Results were given in RLU ± standard deviation (lu µg⁻¹ protein) and, for easier comparison, standardized on the lipofection efficiency of DOTAP-lipoplexes which was set to 100%. (n) number of determinations performed as triplicates. | | | |

## Claims

1. A lipid having the following formula (I) 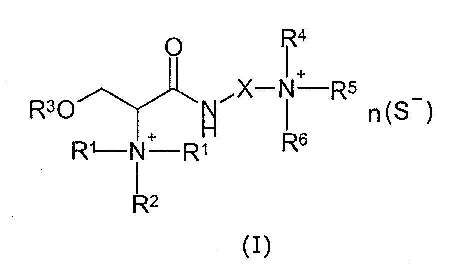 wherein
R¹ are same or different, optionally substituted and/or unsaturated alkyl residues;
R² and R⁶ are independently selected from a free valency, a hydrogen atom and an optionally substituted and/or unsaturated alkyl residue;
R³ is selected from a hydrogen atom, an optionally substituted and/or unsaturated and/or heteroatom-containing alkyl residue, an optionally substituted and/or unsaturated acyl residue, and a suitable protecting group;
R⁴ and R⁵ are independently selected from a hydrogen atom, an optionally substituted and/or unsaturated alkyl residue, an optionally substituted and/or unsaturated acyl residue, an optionally derivatized amino acid or polypeptide residue and a suitable protecting group, or R⁴ and R⁵ together with the adjacent nitrogen atom forms an optionally substituted heterocyclic ring;
X is an optionally substituted and/or unsaturated and/or heteroatom containing alkylene residue;
n(S⁻) represents biologically and/or pharmaceutically suitable counter ion(s).

2. The lipid of claim 1, wherein
(i) R¹ are same or different, optionally substituted and/or unsaturated, linear or branched, non-polar long chain alkyl residues, said alkyl residues preferably having from 6 to 26 carbon atoms; and/or
(ii) R² and R⁶ are independently selected from a free valency, a hydrogen atom, an optionally substituted and/or unsaturated, linear or branched, lower alkyl residue, said lower alkyl residue preferably having from 1 to 6 carbon atoms; and/or
(iii) R³ is selected from hydrogen, an optionally substituted and/or unsaturated and/or heteroatom-containing lower alkyl residue, an optionally substituted and/or unsaturated lower acyl residue, and a hydroxyl protecting group, said lower alkyl residue and said lower acyl residue preferably having from 1 to 6 carbon atoms, and said heteroatoms contained in the lower alkyl residue are preferably selected from O, S and N-R⁷ (where R⁷ is a hydrogen atom or an optionally substituted lower alkyl group; and/or
(iv) R⁴ and R⁵ are independently selected from a hydrogen atom, an optionally substituted and/or unsaturated and/or heteroatom-containing, linear or branched, lower alkyl residue, an optionally substituted and/or unsaturated lower acyl residue (said lower alkyl residue and said lower acyl residue preferably having from 1 to 6 carbon atoms and said heteroatoms contained in the lower alkyl residue are preferably selected from O, S and N-R⁷ (where R⁷ is a hydrogen atom or an optionally substituted lower alkyl group), an amino protecting group, an optionally derivatized amino acid or polypeptide residue, including a residue of formula (II) 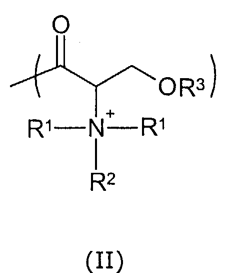 (where all the variables are as defined hereinbefore), and an amino protecting group, R⁴ and R⁵ together with the adjacent nitrogen atom form an optionally substituted, saturated or unsaturated, 5 to 7 membered heterocyclic ring containing 1 to 3 additional heteroatoms selected from N, O and S; and/or
(v) X is an optionally substituted and/or unsaturated and/or heteroatom-containing, linear or branched alkylene residue, said alkylene residue preferably having from 2 to 10 carbon atoms, and said heteroatoms contained in the lower alkyl residue are selected from O, S and N-R⁷ (where R⁷ is a hydrogen atom or an optionally substituted lower alkyl group).

3. The lipid of claim 2, wherein
(i) R¹ is selected from non-polar, optionally substituted, linear or branched, preferably linear C₁₀₋₂₀ alkyl, alkenyl, alkdienyl, alktrienyl and alkinyl residues; and/or
(ii) R² and R⁶ are independently selected from a fee valency, a hydrogen atom, and an optionally substituted, linear or branched C₁₋₃ alkyl residue; and/or
(iii) R³ is selected from a hydrogen atom, a hydrophilic C₁₋₆ alkyl residue, said alkyl residue optionally having hydrophilic substituents or being a polyalkoxyalkyl residue including a residue of the formula -[(CH₂)ᵣO]ₛ-R⁷ (where r is 2 or 3, s is an integer ranging from 1 to 150 and R⁷ is a hydrogen atom or an optionally substituted lower alkyl group), a hydrophilic C₁₋₆ acyl residue optionally having hydrophilic substutents; and/or
(iv) R⁴ and R⁵ are independently selected from hydrogen, an optionally substituted C₁₋₆ alkyl or C₁₋₆ acyl residue optionally having one or more C-C double and/or triple bonds, and an amino protecting group including, but not limited to, carbamate forming groups such as Boc, Troc, and Bn, Tos, allyl, Fmoc, Bz, etc., and a residue of formula (II) 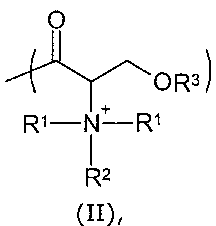 (wherein R¹, R² and R³ are as defined hereinbefore), or
R⁴ and R⁵ may form a 5- to 7-membered heterocyclic ring including, but not limited to, pyrollidino, imidazolino, piperidino, morpholino, azepano, pyridino, etc.; and/or
(v) X is an optionally substituted and/or heteroatom-containing, linear C₂₋₁₀ alkylene residue.

4. The lipid of claim 3, wherein
(i) R¹ is selected from a linear C₁₀, C₁₂, C₁₄, C₁₆, C₁₈ and C₂₀ alkyl and alkenyl residue; and/or
(ii) R² and R⁶ are a free valency, a hydrogen atom or are an optionally substituted linear C₁₋₃ alkyl residue, including, but not limited to, methyl, ethyl and hydroxyethyl; and/or
(iii) R³ is hydrogen, a hydrophilic C₁₋₃ alkyl residue or a hydrophilic C₁₋₃ acyl residue; and/or
(iv) R⁴ is selected from hydrogen, a C₁₋₆ alkyl or C₁₋₆ acyl residue optionally having one C-C double and having hydrophilic substituents, and a residue of formula (II) 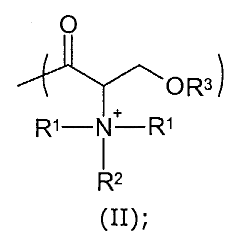 (wherein R¹, R² and R³ are as defined hereinbefore) and/or
(v) R⁵ is hydrogen; and/or
(vi) X is an optionally substituted linear chain C₂₋₁₀ alkylene residue.

5. The lipid of claim 4, wherein
R¹ is selected from C₁₂, C₁₄, C₁₆, C₁₈ alkyl, and oleyl, preferably in case R² is a free valency or a hydrogen atom, R¹ is selected from C₁₂, and C₁₄ alkyl and oleyl, and in case R² is methyl, R¹ is selected from C₁₄, C₁₆ and oleyl;
R² and R⁶ are a fee valency, a hydrogen atom or a methyl group;
R³, R⁴ and R⁵ are hydrogen; and
X is an optionally substituted linear chain C₂₋₆ alkylene residue.

6. A composition for transferring biologically active molecules into cells and/or tissues comprising
(a) at least one lipid as defined in any one of claims 1 to 5; and
(b) at least one biologically active molecule.

7. The composition of claim 6, wherein
(i) the biologically active molecule (b) is selected from the group consisting of nucleic acid constructs, DNA, RNA, synthetic polynucleotides, antisense polynucleotides, missense polynucleotides, nonsense polynucleotides, ribozymes, proteins, peptides, small molecular weight drugs, antibiotics, hormones, etc.; and/or
(ii) the composition further comprises one or more helper compounds/lipids (c) differing from the lipid (a) or a mixture of such helper lipids (c), said helper lipid preferably being selected from phosphatidylcholine, lyso-phospatidylcholine, phosphatidylethanolamine and lyso-phosphatidylethanolamine and the like (having saturated or unsatured carbon chains of variable length); sphingomyelin, ceramide and the like; membrane forming amphiphiles such as block polymers and the like; alkylester, -ether, -amides of sugars, dioles, polyoles, amines, amino acids, peptides and the like; cationic or lipophilized polymers such as DEAE-dextran, histones, polyethylene imine and the like; cholesterol; phosphatidylglyceroles, phosphatidylserines, phosphatidylinositoles and the like; helper agents modulating uptake of lipoplexes by macrophages and other cells such as gangliosides, phosphatidylpolyglycideroles, PEG-phosphatidylethanolamines, proteins (including IgG and Fc-fragments) and the like, preferably the helper lipid is dioleoylphospatidylethanolamine (DOPE) or cholesterol (CHOL); and/or
(iii) the molar ratio of (c)/(a) is from 0.01 to 20, preferably 0.1 to 5, and more preferably is from 0.3 to 1.2 in the case of DOPE and from 0.3 to 1.0, more preferably from 0.3 to 0.7 in the case of CHOL; and/or
(iv) the lipid:DNA charge ratio is 1:1 to 15:1.

8. A process for the manufacture of the lipid as defined in any one of claims 1 to 5 which comprises reacting a compound of formula (III) 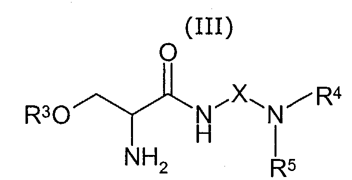 under conditions suitable for carrying out a reductive amination with a compound of formula (IV) 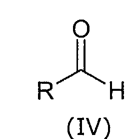 wherein all variables are as defined in claims 1 to 5, and R is defined as R¹ having one CH₂ (methylene) unit less than R¹.

9. Use of the lipid as defined in any one of claims 1 to 5
(i) as constituent of a transfection agent, which optionally further comprises a helper compound/lipid (c) as defined in claim 7, for transferring a biologically active molecule (b) into cells and/or tissues *in vitro,* or
(ii) for preparing an agent including an agent for gene therapy, which optionally further comprises a helper compound/lipid (c) as defined in claim 7, for transferring a biologically active molecule (b) into cells and/or tissues *in vivo.*

10. A method for transferring biologically active molecules into cells and/or into tissues or for gene therapy which method comprises contacting the cells or tissues, or the subject in need of the gene therapy with a composition comprising a cationic lipid of formula (I) as defined in any one of claims 1 to 5.
